## Europäisches Patentamt

## European Patent Office

## Office européen des brevets

(11) Veröffentlichungsnummer: **0 188 470**
**B1**

(12) **EUROPÄISCHE PATENTSCHRIFT**

(45) Veröffentlichungstag der Patentschrift:
25.10.89

(51) Int. Cl.⁴: **A 61 B 3/12,** A 61 F 9/00

(21) Anmeldenummer: 85903228.6

(22) Anmeldetag: 15.07.85

(86) Internationale Anmeldenummer:
PCT/DE 85/00242

(87) Internationale Veröffentlichungsnummer:
WO 86/00512 (30.01.86 Gazette 86/03)

(54) AUGENUNTERSUCHUNGSGERÄT ZUR BETRACHTUNG DES AUGENHINTERGRUNDES.

(30) Priorität: 14.07.84 DE 3425975

(43) Veröffentlichungstag der Anmeldung:
30.07.86 Patentblatt 86/31

(45) Bekanntmachung des Hinweises auf die Patenterteilung:
25.10.89 Patentblatt 89/43

(84) Benannte Vertragsstaaten:
AT BE CH DE FR GB IT LI LU NL SE

(73) Patentinhaber: G. RODENSTOCK INSTRUMENTE GMBH, Drachenseestr. 10 - 12, D-8000 München 70 (DE)

(72) Erfinder: JEAN, Benedikt, Hilbestrasse 40, D-8000 München 19 (DE)
Erfinder: REIS, Werner, Dachauerstr. 47, D-8000 München 50 (DE)

(74) Vertreter: Schiller, Walter, Dr., Kanzlei Münich, Steinmann, Schiller Willibaldstrasse 36- 38, D-8000 München 21 (DE)

(56) Entgegenhaltungen:
DE-A-3 019 477
DE-A-3 130 239
DE-A-3 151 837
FR-A-1 523 270
US-A-3 424 518
US-A-4 423 931

Applied Optics, Band 22, Nr. 12, Juni 1983, New York (US), Seiten 1802-1806 H.D. CRANE et al.: Accurate simulation of visual scotomas in normal subjects", siehe Abbildungen 3,5
Photogrammetric Engineering and Remote Sensing, Band 42, Nr. 6, June 1976, Falls Church, Virginia, Seiten 807-813 G.D. Currie et al.:"Photogrammetric Measurement of the human optic CUP"
New York Journal of Medicine, Band 25, Nr. 18, 15. September 1935, Seiten 901-906 C. Simon et al.: "A

(56) Entgegenhaltungen: (Fortsetzung)
new scientific method of identification"

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Untersuchungsgerät zur Betrachtung des Augenhintergrundes, das eine Einrichtung zur maculanahen Laser-Koagulation der Retina aufweist.

Bei zahlreichen Erkrankungen der Retina werden undichte Gefäßstrecken, neu einsprossende Gefäße sowie unter der Retina liegende Gefäße durch gezielten Beschuß mit Laserstrahlen behandelt. Dabei wird das betreffende Areal durch die vom Laserstrahl deponierte Energie koaguliert.

Der Koagulation-Laserstrahl muß sehr genau auf die krankhaft veränderten Stellen ausgerichtet werden. Ein Auffinden der krankhaft veränderten Stellen ist jedoch nur möglich, sofern diese sichtbar sind. Dies ist aber häufig nicht der Fall. Vielmehr bedürfen diese Areale zu ihrer Lokalisierung einer fluoreszenz-angiographischen Gefäßdarstellung. Das Hauptproblem für den Laserstrahloperateur besteht nun darin, die bei dieser vorausgehenden Untersuchung als behandlungsbedürftig erkannten Areale beispielsweise bei der Beobachtung mittels einer Spaltlampe und einem Kontaktglas auf dem Augenhintergrund wiederzufinden.

Es ist bekannt, daß der Operateur anhand des Photoabzugs eines Angiogramms oder anhand einer anderen mit angiographischen oder sonstigen zweckentsprechenden Methoden gewonnenen Bildvorlage das zu lasernde Areal markiert, sich dieses "merkt" und gedanklich auf den Augenhintergrund überträgt. Dabei orientiert er sich an markanten Strukturen, beispielsweise am Verlauf und Entfernung von Gefäßen oder Gefäßverzweigungen.

In der Praxis hat es sich jedoch als schwierig herausgestellt, diese stereoskopisch angefertigten Aufnahmen des Augenhintergrunds, die beispielsweise auf einem separaten Bildbetrachter direkt an der Spaltlampe montiert sind, gedanklich auf den Augenhintergrund, d. h. auf das reale Fundusbild zu "übertragen". Dies gilt insbesondere im Bereich der Stelle des schärfsten Sehens (Macula), an der wenig Gefäßstrukturen als Orientierungshilfen vorhanden sind. Erschwerend kommt hinzu, daß mit den heute zur Verfügung stehenden Strahlqualitäten ein minimaler Abstand zur Foveola von ca. 100 μm eingehalten werden muß. Wertvolles parazentrales Gesichtsfeld kann verloren gehen, wenn die dem Angiogramm entsprechende Struktur nicht mit der wünschenswerten Sicherheit im Augenhintergrund lokalisiert werden kann.

Der Erfindung liegt die Aufgabe zugrunde, ein Augenuntersuchungs- und Behandlungsgerät zu schaffen, mit dem ohne Schwierigkeiten bei früheren Untersuchungen erkannte krankhafte Veränderungen wiedergefunden werden können.

Diese Aufgabe wird erfindungsgemäß durch die im kennzeichnenden Teil des Anspruchs 1 angegebenen Merkmale gelöst.

Das in den Strahlengang des Untersuchungsgeräts in Richtung auf das Okular des Untersuchungsgeräts eingespiegelte Bild überlagert sich auf dem Fundus für den Beobachter mit dem realen Fundus, so daß beispielsweise vor der Laserbehandlung angiographisch markierte Stellen leicht wiedergefunden werden können.

Durch die Erfindung wird erreicht, daß dem Laseroperateur simultan mit dem Blick auf die Retina das zugehörige Angiogramm zur Verfügung gestellt wird. Das gedankliche Übertragen und die damit verbundene möglichen Fehler entfallen.

Die erfindungsgemäße Ausbildung ist auch zeitsparend, da unmittelbar nach der Fluoreszenz-Angiographie z. B. der Film entwickelt und sofort das Negativ zum Einspiegeln benutzt werden kann. Der wohl bedeutenste Vorteil der Erfindung besteht jedoch darin, daß besonders in der Nähe der Macula, wo das Gesichtsfeld besonders hochauflösend ist, eine hochpräzise Lokalisierung der "Laserherde" erreicht und damit wertvolles Gesichtsfeld erhalten werden kann.

Die erfindungsgemäße Vorrichtung ist natürlich nicht nur zur angiographischen Markierung zu behandelnder Stellen brauchbar, sondern beispielsweise auch zur Verfolgung krankhafter Veränderungen über einen längeren Zeitabschnitt, sowie zum Einspiegeln anderer Informationen und Bilder des Fundus.

Weiterbildungen der Erfindung sind in den Unteransprüchen angegeben:

Das gemäß Anspruch 2 vorgesehene optische System mit variabler Brennweite, beispielsweise ein Zoom-Objektiv oder Recoss-Scheiben, gestattet es, das auf den Fundus projizierte Bild mit dem realen Fundus vollständig zur Deckung zu bringen. Dies ist insbesondere dann wichtig, wenn unterschiedliche Funduskameras zur Herstellung der projizierten Bildvorlagen verwendet werden, oder wenn der Beobachtungsstrahlengang des Augenuntersuchungsgeräts die Möglichkeit bietet, unterschiedliche Vergrößerungen einzustellen.

Die in Anspruch 3 beanspruchte Helligkeitseinstellung des eingespiegelten Bildes unabhängig von der Helligkeitseinstellung beispielsweise einer Spaltleuchte ermöglicht es, einen optimalen Kontrast z. B. eines schwarz/weißen Angiogramms und des farbigen Fundusbildes zu erhalten.

Die in den Ansprüchen 4 und 5 gekennzeichneten Verstellmöglichkeiten erlauben es insbesondere in Verbindung mit der Vergrößerungsänderung des projizierten Bildes, immer ein mit dem Fundus deckungsgleiches projiziertes Bild auf dem Fundus zu erreichen. Die Drehbarkeit um die optische Achse kann beispielsweise bei Verwendung eines Videomonitors als Bildquelle von Bedeutung sein.

Gemäß Anspruch 6 ist ein Betätigungsorgan, beispielsweise ein Fußschalter zum Ein- und Ausblenden des Angiogramms vorgesehen. Dies hat den Vorteil, daß die herkömmliche Durchführung der Laserkoagulation durch das eingespiegelte Angiogramm nicht gestört wird. Solange das Angiogramm eingeblendet ist, wird ein behandlungsbedürftiger Punkt, der auf dem eingespiegelten Angiogramm erkennbar ist, mittels des Zielstrahls des Koagulationslasers "festgehalten". Anschließend wird das Angiogramm ausgeblendet und die Koagulation in üblicher Weise durchgeführt. Nach Wunsch des Operateurs kann aber auch bei eingespiegeltem Fundusbild koaguliert werden.

Als eingespiegelte Bilder eignen sich neben Dia-Negativen und Dia-Positiven auch Videostandbilder, die mittels Videoangiographie gewonnen worden sind. Im Gegensatz zu photographischen Aufnahmen der Gefäßfüllung, von denen maximal etwa 1 Bild/Sek angefertigt werden kann, haben Videoangiogramme den Vorteil, daß wesentlich mehr Bilder pro Zeiteinheit angefertigt werden können, so daß die aussagekräftigste Phase des Kontrastmittelflusses besser ausgewählt werden kann. Ein weiterer Vorteil der Verwendung von Videostandbildern besteht darin, daß sich diese ohne Entwicklung eines Films etc projizieren lassen.

Der Bildträger einschließlich Beleuchtung kann auch austauschbar sein, so daß wahlweise beispielsweise photographische Aufnahmen oder Videostandbilder, die beispielsweise über einen Monitor eingespiegelt werden, als Bildquelle verwendet werden können.

Die erfindungsgemäß vorgesehene Projektionseinrichtung kann in Verbindung mit beliebigen Augenuntersuchungsgeräten, wie Ophthalmoskopen etc verwendet werden. Besonders vorteilhaft ist jedoch die Verwendung zusammen mit einem binokularen Spaltlampengerät (Anspruch 10), da derartige Geräte sehr verbreitet sind.

Aufgrund des einfachen Aufbaus der erfindungsgemäß vorgesehenen Zusatzeinrichtungen ist auch die Nachrüstung an bereits vorhandene Spaltlampengerät ohne weiteres möglich.

Bei Verwendung zusammen mit einer binokularen Spaltlampe ist es von besonderem Vorteil, wenn die Projektionseinrichtung in jeden Beobachtungsstrahlengang ein Bild eines Stereo-Bildpaares des Fundus einspiegelt, da die sterische Betrachtung des Fundus und des deckungsgleich projizierten Stereo-Fundusbildes das Wiederfinden erkrankter Areale sowie das Zielen erleichtert.

In den Ansprüchen 12 bis 14 sind vorteilhafte Ausbildungen des Strahlengangs für die Einspiegelung eines Stereobild-Paares beansprucht, die in einfacher Weise die Anpassung des Basisabstandes der Stereobilder an den Basisabstand des Beobachtungsstrahlenganges erlauben und gleichzeitig zu einem kostengünstigen Aufbau des Geräts führen.

Anspruch 15 gibt eine einfache Möglichkeit an, auch bei einem für sterische Projektion ausgelegtem Gerät mit Monoaufnahmen des Fundus arbeiten zu können.

Die Erfindung wird nachstehend anhand eines Ausführungsbeispiels exemplarisch unter Bezugnahme auf die Zeichnung näher beschrieben, in der zeigen

fig. 1    einen Querschnitt durch eine erfindungsgemäß vorgesehene Projektionseinrichtung, und

fig. 2    einen Längsschnitt durch den Beobachtungsstrahlengang einer Spaltlampe, bei der eine erfindungsgemäße Projektionseinrichtung verwendet wird.

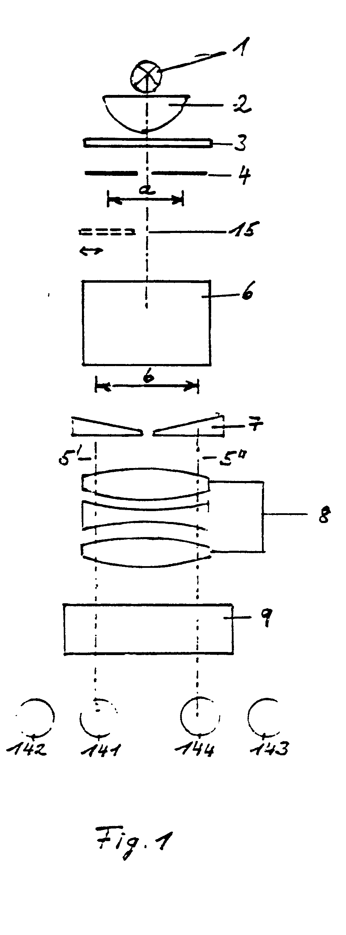

Fig. 1 zeigt eine erfindungsgemäße Projektionseinrichtung im Querschnitt: eine Halogenlampe 1 beleuchtet über einen Kondensor 2 und ein Mattglas 3 eine Filmebene 4.

Das in der Filmebene 4 angeordnete Stereobild, beispielsweise ein Stereo-Angiogramm, wird über ein für beide Strahlengänge 5' und 5" gemeinsames Objektiv 6, ein Glaskeil-Paar 7 und ein für beide Strahlengänge gemeinsames Variooobjektiv 8 auf einen Teiler-Umlenkspiegel 9 projiziert.

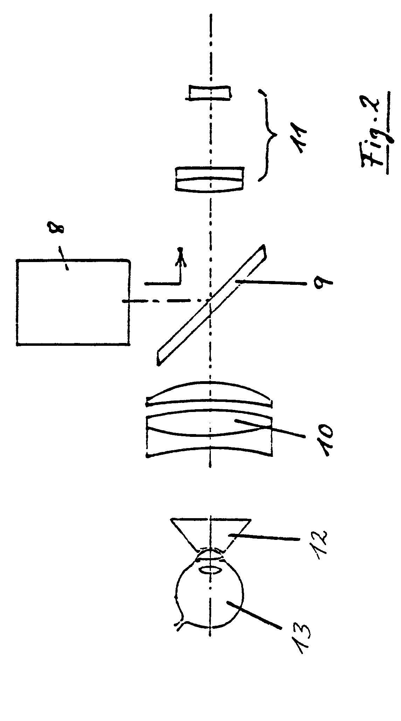

Fig. 2 zeigt, daß der Teiler-Umlenkspiegel 9 das projizierte Stereobild in den Beobachtungs-Strahlengang einer (binokularen) Spaltlampe einspiegelt. Der Beobachtungs-Strahlengang der Spaltlampe weist ein Frontobjektiv 10, einen Galilei-Vergrößerungswechsler 11 zum Einstellen verschiedener Vergrößerungen und ein Drei-Spiegel-Kontaktglas 12 auf, das auf das zu untersuchende Auge aufgesetzt ist. (In der Zeichnung ist der Strahlengang in Seitenansicht dargestellt)

Das in den Figuren 1 und 2 dargestellte Augenuntersuchungsgerät arbeitet wie folgt:

Die Elemente 1 bis 3 projizieren das Stereobild, das in der Filmebene 4 angeordnet ist, über die optischen Elemente 6 bis 8 in den eigentlichen Strahlengang des Untersuchungsgeräts, der von den Elementen 10 bis 12 gebildet wird. Unabhängig von der im Strahlengang des Untersuchungsgeräts eingestellten Vergrößerung läßt sich das projizierte Bild immer durch Verstellen der Vergrößerung des Varioobjektivs (Zoomobjektivs) 8 mit dem Augenhintergrund des Auges 13 zur Deckung bringen.

Das Glaskeil-Paar 7 dient dabei dazu, den Basisabstand des Stereobild-Paares dem Basisabstand des Stereo-Beobachtungsstrahlenganges anzupassen. Beispielsweise beträgt der Basisabstand a einer Zeiss-Stereofunduskamera, die auf 24 * 36 mm Film aufzeichnet, 18 mm, während der Basisabstand b z. B. der Rodenstock-Spaltleuchte Ro 2000 S 24 mm beträgt. Der Keilwinkel der Keile 7 beträgt typischerweise zwischen 4 bis 5 Grad. Zur Anpassung der Basisabstände von verschiedenen Funduskameras und Spaltleuchten ist bei dem gezeigten Ausführungsbeispiel das Glaskeil-Paar 7 austauschbar, so daß die Aufnahmen nahezu beliebiger Stereo-Funduskameras mit beliebigen Spaltleuchten kombiniert werden können.

Das Glaskeil-Paar 7 erzeugt aus den Strahlengängen 5' und 5" insgesamt vier Teilbilder 141 bis 144 (zwei rechte Bilder 141, 143 und zwei linke 142, 144), von denen das rechte Teilbild 143 und das linke Teilbild 142 im Beobachtungsstrahlengang (Okulargesichtsfeld) der Spaltleuchte nicht sichtbar sind.

Bei dem gezeigten Ausführungsbeispiel weist das Objektiv 6 eine Brennweite von 286 mm auf, die zu einer Vergrößerung von 2,2 führt. Die Brennweite des Vario-Objektiv 8 ist zwischen 221 und 351 mm verstellbar, die Vergrößerung beträgt ± 0,5.

Vorstehend ist die Erfindung anhand eines Ausführungsbeispiels beschrieben worden. Im Rahmen des allgemeinen Erfindungsgedankens, ein Bild des Fundus und insbesondere ein Angiogramm simultan und deckungsgleich mit dem Augenhintergrund in den Beobachtungsstrahlengang in Richtung auf das Okular des Geräts einzuspiegeln, sind die verschiedensten Modifikationen möglich:

Das Glaskeil-Paar kann zusätzlich oder anstelle der Austauschbarkeit auch längsverschiebbar sein, um den Basisabstand des Stereobild-Paares an den Abstand der Stereo-Beobachtungsstrahlengänge anzupassen.

Der Bildträger 4 und die Beleuchtungsquelle 1 - 3 können austauschbar sein, so daß anstelle des photographischen Films z. B. auch ein Mikro-Monitor, der das Angiogramm als Videostandbild wiedergibt, als Bildquelle verwendet werden kann.

Ferner kann in einem der beiden Stereo-Projektionsstrahlengänge eine Blende 15 vorgesehen werden, die bei Verwendung einer Monoaufnahme anstelle eines Stereo-Bildpaares einschwenkbar ist. Damit können auch Monoaufnahmen in Verbindung mit Stereo-Spaltleuchten verwendet werden.

Selbstverständlich ist es auch möglich, ein Gerät mit rein monokularem Beobachtungsstrahlengang in Verbindung mit der erfindungsgemäß vorgesehenen Projektionseinrichtung zu verwenden.

In jedem Falle hat das erfindungsgemäße Augenuntersuchungsgerät mit Simultaneinspiegelung einer Fundusaufnahme und insbesonder eines Angiogramms eine Reihe von Vorteilen: Das Gerät eignet sich beispielsweise zur makulanahen Laser-Photokoagulation mit dem roten Krypton-Laser bei seniler disciformer Maculopathie und zur exakten Lokalisierung paramaculärer Leckagepunkte bei Retinitis centralis serosa. Auch neu aufgetretene paramaculäre Mikroaneurysmen oder Punktblutungen bei Retinopathia diabetica lassen sich durch Superposition älterer Angiogramme mit dem aktuellen Fundusbefund rasch erkennen.

**Patentansprüche**

1. Augenuntersuchungsgerät zur Betrachtung des Augenhintergrundes, mit einer Einrichtung zur maculanahen Laser-Koagulation der Retina, dadurch gekennzeichnet, daß eine Projektionseinrichtung (1, 2, 3, 6, 7, 8) vorgesehen ist, die in den Beobachtungsstrahlengang des Untersuchungsgeräts mittels eines Strahlteilers (9) ein Bild und insbesondere ein Angiogramm des Augenhintergrundes des zu untersuchenden bzw. zu behandelnden Auges (13) in Richtung auf das Okular (11) des Untersuchungsgeräts einspiegelt, so daß der Beobachter das eingespiegelte Bild mit dem Bild des realen Augenhintergrunds überlagern kann.

2. Gerät nach Anspruch 1, dadurch gekennzeichnet. daß die Projektionseinrichtung ein optisches System (8) mit variabler Brennweite aufweist.

3. Gerät nach einem der Ansprüche 1 oder 2, dadurch gekennzeichnet, daß die Helligkeit des eingespiegelten Bildes einstellbar ist.

4. Gerät nach einem der Ansprüche 1 bis 3, dadurch gekennzeichnet, daß das eingespiegelte Bild um seine optische Achse drehbar ist.

5. Gerät nach Anspruch 4, dadurch gekennzeichnet, daß zur Drehung des eingespiegelten Bildes um seine optische Achse der Bildträger (4) der Projektionseinrichtung drehbar ist.

6. Gerät nach einem der Ansprüche 1 bis 5, dadurch gekennzeichnet, daß ein Betätigungsorgan zum Ein- und Ausblenden des eingespiegelten Bildes vorgesehen ist.

7. Gerät nach einem der Ansprüche 1 bis 6, dadurch gekennzeichnet, daß das eingespiegelte Bild ein auf einem Film aufgezeichnetes Bild des Augenhintergrundes ist.

8. Gerät nach einem der Ansprüche 1 bis 7, dadurch gekennzeichnet, daß das eingespiegelte Bild ein Videostandbild ist.

9. Gerät nach einem der Ansprüche 1 bis 8, dadurch gekennzeichnet, daß das Untersuchungsgerät eine binokulare Beobachtung des Augenhintergrundes erlaubt.

10. Gerät nach Anspruch 9, dadurch gekennzeichnet, daß das Gerät eine binokulare Spaltlampe ist.

11. Gerät nach Anspruch 9 oder 10, dadurch gekennzeichnet, daß die Projektionseinrichtung in jeden Beobachtungsstrahlengang jeweils ein Bild eines Stereobildpaares einspiegelt.

12. Gerät nach Anspruch 11, dadurch gekennzeichnet, daß die beiden Projektions-Strahlengänge (5', 5") ein gemeinsames optisches System (8) mit variabler Brennweite und ein Glaskeil-Paar (7) aufweisen.

13. Gerät nach Anspruch 12, dadurch gekennzeichnet, daß zur Anpassung des Basisabstandes (a) der Stereobilder an den Basisabstand (b) des binokularen Beobachtungsstrahlengangs das Glaskeil-Paar (7) austauschbar ist.

14. Gerät nach Anspruch 12 oder 13, dadurch gekennzeichnet, daß zur Anpassung des Basisabstandes (a) der Stereobilder an den Basisabstand (b) des binokularen Beobachtungsstrahlengangs das Glaskeil-Paar (7) in Richtung der optischen Achse der Projektionseinrichtung verschiebbar ist.

15. Gerät nach einem der Ansprüche 10 bis 14, dadurch gekennzeichnet, daß in einen Strahlengang (5') der Projektionseinrichtung eine Blende (15) einschwenkbar ist, so daß auch Mono-Bilder einspiegelbar sind.

## Claims

1. Ophthalmoligcal examination instrument for viewing the fundus, with a device for the laser coagulation of the retina in the macular region, characterised by the fact that a projection device (1, 2, 3, 6, 7, 8) is provided which projects an image and, in particular, an angiograph of the fundus of the eye (13) to be examined and/or treated into the viewing beam path of the examination unit in the direction to the eyepiece (11) of the examination unit by means of beam splitter (9) so that the observer can superimpose the projected image onto the image of the real fundus.

2. Instrument according to claim 1, characterised by the fact that the projection device possesses an optical system (8) with variable focal length.

3. Instrument according to claims 1 or 2, characterised by the fact that the brightness of the image projected can be adjusted.

4. Instrument according to any of the claims 1 to 3, characterised by the fact that the image projected can rotated around its optical axis.

5. Instrument according to claim 4, characterised by the fact that to rotate the projected image around its optical axis, the picture carrier (4) of the projection device can be rotated.

6. Instrument according to any of the claims 1 to 5, characterised by the fact that an activating device is provided for the fading in or out of the image projected.

7. Instrument according to any of the claims 1 to 6, characterised by the fact that the image projected is an image captured on film.

8. Instrument according to any of the claims 1 to 6, characterised by the fact that the image projected is a video still.

9. Instrument according to any of the claims 1 to 8, characterised by the fact that the examination instrument permits binocular viewing of the fundus.

10. Instrument according to claim 9, characterised by the fact that the instrument is a binocular split-lamp.

11. Instrument according to claims 9 or 10, characterised by the fact that the projection device projects one image of a stereo image pair into each viewing beam path.

12. Instrument according to claim 11, characterised by the fact that the two projection beam paths (5', 5") have a common optical system (8) with variable focal length and a glass prism pair (7).

13. Instrument according to claim 12, characterised by the fact that to adapt the base distance (a) of the stereo images to the base distance (b) of the binocular viewing beam path, the glass prism pair (7) is exchangeable.

14. Instrument according to claims 12 or 13, characterised by the fact that to adpat the base distance (a) of the stereo images to the base distance (b) of the binocular viewing beam path, the glass prism pair (7) can be moved in the direction of the optical axis of the projection

15. Instrument according to any of the claims 10 to 14, characterised by the fact that a mask (15) can be swung into a beam path (5') of the projection device so that also mono images can be projected.

## Revendications

1. Appareil d'examen des yeux pour observer le fond de l'oeil avec un dispositif permettant la coagulation par laser de la rétine à proximité immédiate de la macula, caractérisé par le fait qu'un dispositif de projection (1, 2, 3, 6, 7, 8) qui réfléchit au moyen d'une lame séparatrice (9) une image dans la marche des rayons d'observation de l'appareil d'examen et en particulier un angiogramme du fond de l'oeil (13) à examiner respectivement à soigner en direction de l'oculaire (11) est prévu, de sorte que l'observateur puisse superposer l'image réfléchie à l'image du fond de l'oeil réelle.

2. Appareil selon la revendication 1, caractérisé par le fait que le dispositif de projection présente un système optique (8) à distance focale variable.

3. Appareil selon la revendication 1 ou 2, caractérisé par le fait que la luminosité de l'image réfléchie est réglable.

4. Appareil selon une quelconque des revendications 1 à 3, caractérisé par le fait que l'image réfléchie est orientable autour de son axe optique.

5. Appareil selon la revendication 4, caractérisé par le fait que pour réaliser la rotation de l'image réfléchie autour de son axe optique le porteur d'images (4) du dispositif de projection est orientable.

6. Appareil selon une quelconque des revendications 1 à 5, caractérisé par le fait qu'un organe de mise en action pour ouvrir en fondu et pour diaphragmer l'image réfléchie est prévu.

7. Appareil selon une quelconque des revendications 1 à 6, caractérisé par le fait que l'image réfléchie représente une image du fond de l'oeil enregistrée sur film.

8. Appareil selon une quelconque des revendications 1 à 7, caractérisé par le fait que l'image réfléchie est une image vidéo immobile.

9. Appareil selon une quelconque des revendications 1 à 8, caractérisé par le fait que l'appareil d'examen permet l'observation binoculaire du fond de l'oeil.

10. Appareil selon la revendication 9, caractérisé par le fait que l'appareil est une lampe à fente binoculaire.

11. Appareil selon la revendication 9 ou 10, caractérisé par le fait que le dispositif de projection réfléchit dans chaque marche des rayons d'observation respectivemant une paire d'images stéréoscopiques.

12. Appareil selon la revendication 11, caractérisé par le fait que les deux marches des rayons de projection (5', 5") présentent un système optique commun (8) à distance focale variable et une paire de coins sensitométriques (7).

13. Appareil selon la revendication 12, caractérisé par le fait que pour l'adaptation de la distance de base (a) des images stéréoscopiques à la distance de base (b) de la marche des rayons d'observation binoculaire la paire de coins sensitométriques (7) est échangeable.

14. Appareil selon la revendication 12 ou 13, caractérisé par le fait que l'adaptation de la distance de base (a) des images stéréoscopiques à la distance de base (b) de la marche des rayons d'observation binoculaire la paire de coins sensitométriques (7) est déplaçable en direction de l'axe optique du dispositif de projection.

15. Appareil selon une quelconque des revendications 10 à 14, caractérisé par le fait qu'il est possible d'orienter dans une marche des rayons (5') du dispositif de projection un diaphragme (15) ce qui permet la reflexion de monoimages.

Fig. 1

_Fig.2_